# EUROPÄISCHE PATENTSCHRIFT

(11) **EP 0 319 604 B1**
(45) Veröffentlichungstag und Bekanntmachung des Hinweises auf die Patenterteilung: **29.09.1993**
(21) Anmeldenummer: 87118213.5
(22) Anmeldetag: 09.12.1987
(51) Int. Cl.: C12M 1/34, G01N 33/18

(54) **Biotechnischer Monitor zur kontinuierlichen Bestimmung von Schadwirkungen auf Mikroorganismen in Wasser**
Biotechnical monitor for the continuous determination of toxin effects on micro-organisms in water
Moniteur biotechnique pour la détermination continue des effets nocifs sur les micro-organismes dans l'eau

(43) Veröffentlichungstag der Anmeldung: 14.06.1989
(73) Patentinhaber: Reuter, Wilhelm A., D-56112 Lahnstein (DE); Nöthlich, Ingo, Dr., D-56073 Koblenz (DE)
(72) Erfinder: Reuter, Wilhelm A., D-56112 Lahnstein (DE); Nöthlich, Ingo, Dr., D-56073 Koblenz (DE)

(56) Entgegenhaltungen:
- DE-A- 2 513 650
- US-A- 4 162 195
- US-A- 4 330 385

## Beschreibung

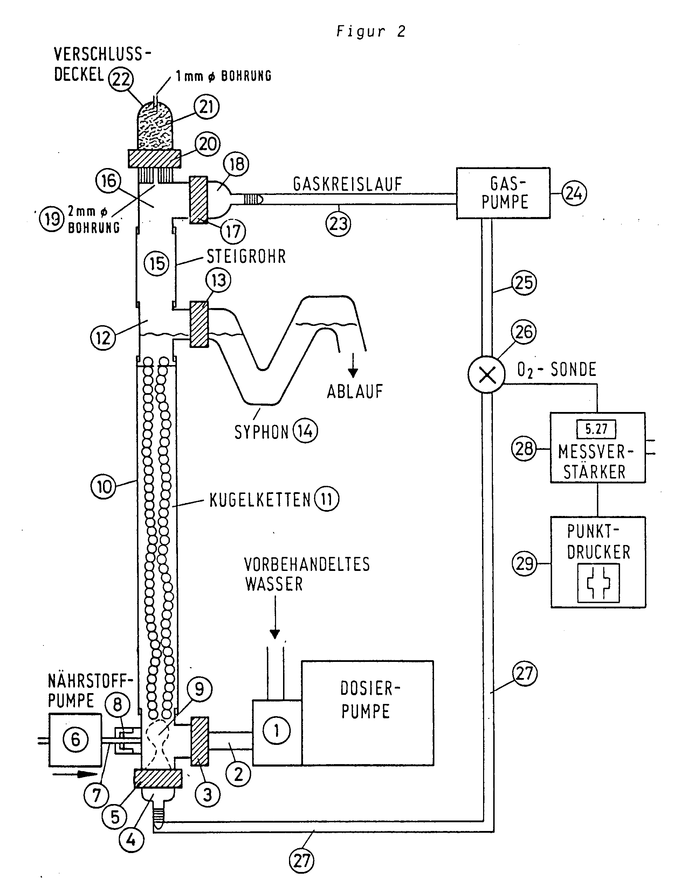

Die Erfindung stellt einen kontinuierlich arbeitenden Wirkungstest in Wasser dar. Erfindungsgemäß besteht der biotechnische Monitor aus zwei speziellen Biodetektoren (Fig.2), in denen jeweils ein Bakterienrasen unter definierten Nährstoff- und Temperaturbedingungen über unbegrenzte Zeit gehalten wird. Jeder Biodetektor wird wechselweise mit 34 Grad Celsius temperiertem und bis zur Sauerstoffsättigung angereichertem Testwasser bzw. aufbereitetem Wasser (zur Regeneration) beschickt (1).Ein starker,im Kreislauf geführter Gasstrom (23,24,25,27) erzeugt durch mechanischen Abrieb eine dünne gleichmäßig aktive Bakterienschicht auf den Aufwuchsflächen ( 10 , 11 ) des Biodektors, deren Sauerstoffverbrauch sich mit dem Zustrom von Sauerstoff aus dem Wasser und über den Druckausgleich mit der Atmosphäre in dynamischem Gleichgewichtszustand befindet. Der Druckausgleich erfolgt über eine 2mm-Bohrung (19) und eine 1mm-Bohrung in der Verschlußkappe (22), welche zur Verlangsamung des Druckausgleiches mit Mineralwolle (21) gestopft ist. Der Sauerstoffgehalt wird verschmutzungsfrei mit der Sauerstoffsonde (26) im Gaskreislauf gemessen. Der dynamische Gleichgewichtszustand im Biodetektor wird über die Nährstoffmenge, die steril und hochkonzentriert zugegeben wird (6,7,8), bei ca 50% Sauerstoffsättigung im Gasstrom geregelt, wodurch stimulierende oder hemmende Wirkungen des Testmediums als Veränderung der Gleichgewichtslage angezeigt werden (26,28,29).

Die Erfindung bezieht sich auf einen kontinuierlich arbeitenden biotechnischen Monitor, der aus der Kombination von zwei Biodetektoren besteht, in welchen je ein Rasen aus Mikroorganismen unter optimalen, steuerbaren Bedingungen über unbegrenzte Zeit gehalten und die Veränderungen der mikrobiellen Atmungsaktivität durch das zu testende Wasser über den Sauerstoffgehalt in der Gasphase bestimmt wird.
Mikroorganismen in Biotests sind ein geeignetes Mittel für eine summarische Erfassung und Überwachung gefährlicher Stoffe. Innerhalb der Fachdiskusion weist die einzuschlagende Meßstrategie derzeit in zwei Richtungen:
a) Substanz - Screening zur Ermittlung toxikologischer Kennwerte
b) Kontrollen zum Schutz eines Ökosystems z.B. Fließgewässer oder spezieller Einrichtungen wie Kläranlagen und Trinkwassergewinnung
Während zu Punkt a) ein vielfältiges und breites Spektrum an Tests zur Verfügung steht ist die Entwicklung auf dem Sektor b) noch nicht weit gediehen.

Wichtigste Vorraussetzung für ein geeignetes Überwachungskonzept sind die Erfassung von Schadwirkungen im subletalen Konzentrationsbereich sowie die kontinuierliche Messung der zu überwachenden Wässer bei vertretbarem Wartungsaufwand.

Diese Voraussetzungen werden durch die Erfindung, wie sie in den Ansprüchen gekennzeichnet ist, erfüllt. Der biotechnische Monitor besteht aus zwei unabhängig voneinander arbeitenden Biodetektoren, deren Biorasen durch mechanischen Abrieb eines im Kreislauf geführten Gasgemisches, geeigneter Temperaturbedingungen und Nährstoffzufuhr optimale Atmungsaktivität aufweist. Beide Biodetektoren werden wechselweise mit Testwasser und aufbereitetem Wasser ( z.B. Trinkwasser ) kontinuierlich beschickt. Durch diesen zyklichen Wechsel wird der Aktivitätszustand der Biorasen kontrolliert und falls erforderlich, in dem Reinwasserzyklus regeneriert.
Durch diese Anordnung ergibt sich für den, sich ständig erneuernden Biorasen eine im Betrieb praktisch unbegrenzte Standzeit, sofern keine Totalvergiftung der Biologie eintritt.

Die Anordnung zur Sauerstoffmessung, welche verschmutzungsfrei in der Gasphase erfolgt, hat eine Standzeit von mehr als einem halben Jahr.
Durch die hohe Konzentration des Nährmediums, welches nur sehr langsam zudosiert wird, muß der Nährstoffvorrat nur alle 14 Tage ergänzt werden.
Der Wartungsaufwand ist somit gering. Der Biomonitor ist in seinen Bauteilen auf lange Lebensdauer und Standzeit optimiert.

Neben dieser für kontinuierliche Biotests mit Mikroorganismen ungewöhnlich hohen Standzeit ist die Empfindlichkeit gegenüber Schadstoffen hervorzuheben. Schon kleinste Mengen z.B. 0,3 mg Kupfersulfat können nach nur 3 - 5 min einwandfrei bestimmt werden. Diese sich durch die Erfindung ergebenden Vorteile ermöglichen die kontinuierliche Überwachung von Oberflächengewässern, den vorsorglichen Schutz von Trinkwassergewinnungsanlagen,die Beurteilung von industriellen Einleitungen, die Kontrolle von Kläranlagen ( Einlauf, Auslauf ) sowie die Sickerwasserkontrolle bei Mülldeponien.

DE-A-2 513 650 beschreibt eine Vorrichtung und ein Verfahren zur kontinuierlichen Eichkontrolle eines Toximeters. Dazu werden in einer Apparatur fortwährend Bakterien gezüchtet, die, mit Probenflüssigkeit vermischt, einer Meßzelle zugeführt werden, wo der Sauerstoffgehalt in der flüssigen Phase gemessen wird, der als Maß für die Toxizität der Probenflüssigkeit benutzt wird.

US-A-4 162 195 offenbart ein Verfahren zur Untersuchung von Abwasserproben, wobei eine mit Sauerstoff angereicherte Probe mit einer Mikroorganismenpopulation vermischt wird. Anschließend wird der Sauerstoffgehalt in der flüssigen Phase gemessen. Ein zu hoher Wert gilt dabei als Zeichen für das Vorhandensein von toxischen Substanzen in der Probe.

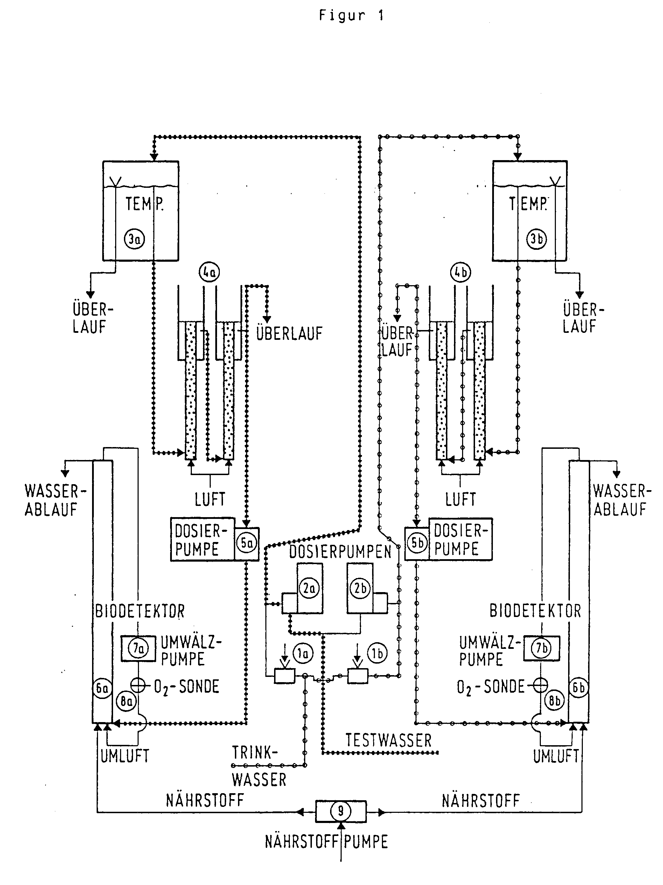

Die Erfindung wird an Hand eines Ausführungsbeispiels naher erläutert. Figur 1 zeigt die beiden Biodetektoren des Biomonitors in schematischer Darstellung. Über die Magnetventile(1a) und (1b) werden die Biodetektoren mit gereinigtem Wasser und über die Dosierpumpen (2a) und (2b) mit Testwasser versorgt, wobei das Ventil (1a) mit der Dosierpumpe (2b) und Ventil(1b) mit der Dosierpumpe(2a) so geschaltet ist, daß das Ventil (1a) nur dann geöffnet ist, wenn die Dosierpumpe (2b) in Betrieb ist, während zu dieser Zeit das Ventil (1b) und die Dosierpumpe (2a) geschlossen sind und umgekehrt. Durch diese Art der Schaltung werden die Wässer wechselweise über je eine gemeinsame Transportleitung den Temperiereinheiten (3a) und (3b) zugeführt.
Die Temperiereinheiten regeln die Wassertemperatur auf einen Wert von 34 Grad Celsius. Die Temperiereinheiten verfügen über Überläufe, die zu große Wassermengen ableiten und über Abläufe,die das Wasser von der Oberfläche her zu den Belüftungeinheiten(4a) und (4b) in freiem Gefälle führen.
Die Belüftungseinheiten bestehen aus je zwei Belüftungsstrecken, die hintereinander geschaltet sind. Die Belüftungsstrecken werden von unten nach oben von dem zugeführten Wasser und feinperliger Luft durchströmt. Die Länge der Belüftungsstrecken ist so gewählt, daß immer eine Sauerstoffsättigung des Wassers erreicht wird. Am oberen Ende der Belfüftungsstrecken erfolgt die Trennung von Gas- und Wasserphase. Die Luft kann über das offene Rohrende entweichen,während das Wasser aufgefangen und weiter zu den Dosierpumpen (5a) und (5b) geleitet wird. Zu große Wassermengen können durch einen Überlauf in der zweiten Belüftungsstrecke abfließen. Die Dosierpumpen sind so eingestellt, daß sie jeweils 0,5 Liter Wasser pro Minute in die Detektorsäulen (6a)und(6b) drücken.
Das Wasser durchfließt die jeweilige Detektorsäule mit Nährstoffen angereichert ( über Nährstoffpumpe 9 ) zusammen mit einem starken, turbulenten Gasstrom von unten nach oben. Das Wasser verläßt in den Säulenköpfen Ober Syphons die Detektorsäulen, während die Gasströme zurückgeführt und von Gaspumpen (7a) und (7b) umgewälzt werden.
In den Gasströmen, auf der Druckseite der Gaspumpen, wird jeweils der Sauerstoffgehalt durch Sauerstoffsonden (8a) und (8b) gemessen. Figur 2 zeigt den erfindungsgemäßen Aufbau eines Biodetektors. Hierbei sind die Dosierpumpe (1), die das temperierte und belüftete Wasser fördert, über die Schlauchverbindung (2) und die Verschraubung (3), die Nährstoffpumpe (6) über den Schlauch (7) und die Verschraubung (8),sowie der im Kreis geführte Gasstrom auf seiner Druckseite (27),über den Schlauchanschluß (4) und die Verschraubung (5) an den Fuß (9) der Detektorsäule (10) angeschlossen. In der senkrecht stehenden Detektorsäule (10) sind die, als zusätzliche Aufwuchsfläche dienenden Kugelketten (11) befestigt. Die Kugelketten sind so an ihrem oberen Ende befestigt, daß sie frei und beweglich in der Detektorsäule hängen.
Das von der Dosierpumpe (1) geförderte Wasser steigt durch die Detektorsäule an den Kugelketten vorbei bis zu dem T-Stück (12) am Ende der Detektorsäule(10). Dort wird es von dem ebenfalls von unten nach oben in großen Gasblasen aufsteigenden Gasstrom getrennt und verläßt die Detektorsäule über die Verschraubung (13) und den Syphon (14). In dem Steigrohr (15) wird der Gasstrom von dem mitgerissenen Wasser getrennt und über das T-Stück(16), die Verschraubung (17), den Gasanschluß(18) und den Schlauch (23) zur Gaspumpe(24) geführt. Von der Druckseite der Gaspumpe wird der Gasstrom durch die Schlauchverbindung (25) zur Sauerstoffsonde (26) geleitet. Hier wird der Sauerstoffgehalt im Gasstrom gemessen und das Meßsignal über Kabel zum Meßverstärker (28) weiter gegeben. Der Meßwertverstärker bildet das Meßsignal digital ab und steuert über Mini-Max-Relais Warn- und Probenahmegeräte. Weiter wird der verstärkte Meßwert zu einem Zwei-Kanal-Punkt-Drucker (29) geführt und dort registriert.
Der Gasstrom wird von der Sauerstoffsonde (26) über eine weitere Schlauchverbindung (27) zurück zum Schlauchanschluß(4) am Fuß der Detektorsäule geleitet. Der Druckausgleich mit der Atmosphäre erfolgt über eine Bohrung von 2 mm im scheibenförmigen Verschluß (19) der oberen Öffnung des T-Stückes (16) und einer weiteren Bohrung von 1 mm in der Mitte der Abschlußkuppel der Verschlußkappe (22).Die Verschlußkappe(22) ist zur Verlangsamung des Druckausgleichs mit Mineralwolle (21) gestopft und mit der Verschraubung (20) an das T-Stück(16) angeschlossen.

## Patentansprüche

1. Kontinuierliches Verfahren zur Untersuchung von Wasser durch Feststellung der Schadwirkung darin enthaltener Schadstoffe auf die Atmungsaktivität von Mikroorganismen, wobei das zu testende Wasser zuerst temperiert und in einer Belüftungseinheit mit Sauerstoff gesättigt wird, dann zusammen mit einem im Kreis geführten Gasstrom und Nährstoffen von unten durch eine Detektorsäule geleitet wird, die eine gleichmäßig aktive Schicht von Mikroorganismen enthält, dadurch gekennzeichnet, daß anschließend die flüssige Phase von der Gasphase getrennt wird, und die Gasphase mit einer Gaspumpe, zum Fuß der Detektorsäule zurückgeleitet wird, wobei der 02-Gehalt der Gasphase auf der Druckseite der Pumpe durch eine Sauerstoffsonde gemessen und der Meßwert registriert wird.

2. Kontinuierliches Verfahren nach Anspruch 1, dadurch gekennzeichnet, daß die Mikroorganismen-Schicht in der Detektorsäule durch einen starken, turbulenten Gasstrom mechanisch so belastet wird, daß ein dünner, gleichmäßig aktiver Biorasen entsteht.

3. Kontinuierliches Verfahren nach den Ansprüchen 1-2, dadurch gekennzeichnet , daß die Nährstoffe steril und in konzentrierter Form zugegeben werden.

4. Kontinuierliches Verfahren nach den Ansprüchen 1-3, dadurch gekennzeichnet, daß die Detektorsäule wechselweise mit auf 34 Grad Celsius temperiertem und bis zur Sättigung mit Sauerstoff angereichertem Testwasser und aufbereitetem Wasser ( z.B. Trinkwasser) beschickt wird.

5. Kontinuierliches Verfahren nach den Ansprüchen 1-4, dadurch gekennzeichnet, daß sich der Sauerstoffverbrauch der Mikroorganismen-Schicht durch den Zustrom von Sauerstoff im Testmedium und den Druckausgleich mit der Atmosphäre in einem dynamischen Gleichgewichtszustand befindet.

6. Kontinuierliches Verfahren nach Anspruch 4, dadurch gekennzeichnet, daß der dynamische Gleichgewichtszustand über die Nährstoffmenge bei etwa 50% der Sauerstoffsättigung eingestellt wird, wodurch stimmulierende oder hemmende Wirkungen des Testmediums als Veränderung der Gleichgewichtslage direkt angezeigt wird.

7. Biomonitor zur kontinuierlichen Untersuchung von Wasser durch Feststellung der Schadwirkung darin enthaltener Schadstoffe auf die Atmungsaktivität von Mikroorganismen, enthaltend
Temperiereinheiten und Belüftungseinheiten zur Sauerstoffsättigung des zu testenden Wassers und zur Einstellung der für Bakterien optimalen Temperatur,
2 wechselseitig schaltbare Detektorsäulen, die eine gleichmäßig aktive Schicht von Mikroorganismen enthalten,
Vorrichtungen zum Einleiten des vorbehandelten Wassers am Fuße der Detektorsäulen,
Vorrichtungen zur Einleitung eines starken, turbulenten Gasstromes am Fuß der Detektorsäulen,
dadurch gekennzeichnet, daß Vorrichtungen zur Abtrennung der flüssigen Phase von der Gasphase am Kopf der Detektorsäulen vorhanden sind, sowie Schlaucheinrichtungen um den abgetrennten Gasstrom im Kreis zu führen, in deren Verlauf eine Gaspumpe und eine Sauerstoffsonde zur Messung des 02-Gehaltes des Gasstromes angebracht sind.

8. Biomonitor nach Anspruch 7, dadurch gekennzeichnet, daß der Druckausgleich mit der Atmosphäre über zwei Bohrungen von 1mm und 2mm Durchmesser im Verschlußdeckel des Steigrohres am oberen Ende der Detektorsäulen erfolgt.

9. Biomonitor nach den Ansprüchen 6-7 dadurch gekennzeichnet, daß der Raum zwischen den beiden Bohrungen im Verschlußdeckel der Detektorsäulen mit Mineralwolle oder vergleichbarem Material so gestopft ist, daß nur ein langsamer Druckausgleich zwischen dem Detektorinneren und der Atmosphäre erfolgen kann.

## Claims

1. The biotechnical monitor for the continuous determination of toxin effect on micro - organisms in water.
The process in the biotechnical monitor is characterised by warming up the water to be tested and saturising it with oxygen afterwards.In the following it is sent together with a circulating stream of air and nutriants through a detecting tube from below,which contains an evenly active layer of micro - organisms.After that process the liquid is seperated from the air and the air is sent back to the bottom of the detecting tube with the help of a pump.The concentration of oxygen is measured in the air.

2. The biotechnical monitor for the continuous determination of toxin effect on micro - organisms in water,
by the fact the layer of micro - organisms is mechanically stessed by a strong stream of air in that way,than a thus and evenly active layer can evolve.

3. The biotechnical monitor for the continuous determination of toxin effect on micro - organisms in water(look at 1.,2.), the nutriants can be added in a sterile and concentrated form.

4. The biotechnical monitor for the continuous determination of toxin effect on micro - organisms in water( look at 1., 2.,3.),
that the test tube can be charged alternatively with the test water and the drinking water.

5. The biotechnical monitor for the continuous determination of toxin effect on micro - organisms in water (look at 1., 2.,3.,4.),
that the consumption of the oxygen and the suppley of oxygen are balanced.

6. The biotechnical monitor for the continuous determination of toxin effect on micro - organisms in water(look at 4.), the dynamic balance is reached at about 50%, where changes of the balance are indicated.

7. The biotechnical monitor for the continuous determination of toxin effect on micro - organisms in water,
the process contains:
devices to warm up, to saturate the test water with oxygen two test tubes which can be turned on alternatively devices to fill in the prepared water at the bottom of the test tube.
devices to introduce the air to the bottom of the test tube
devices to separate the liquid from the air at the top of the tube; in this air the concentration of oxygen is measured.

8. The biotechnical monitor for the continuous determination of toxin effect on micro - organisms in water (look at 7), the process contains
devices that the adjustment of preesure is reached via the bores of 2 mm and 1 mm in the lid of the tube.

9. The biotechnical monitor for the continuous determination of toxin effect on micro - organisms in water (look at 6., 7.), the process contains
devices that the glass wool in the lid guaranties a slow adjustment of pressure.

## Revendications

1. Procédé continu pour l'analyse d'eau, déterminant l'effet nuisible de substances nocives y contenues sur l'activité respiratoire de microorganismes; l'eau à tester est d'abord tempérée et saturée d'oxygène dans une unité d'aération, transportée ensuite, en même temps qu'un courant de gaz mene en circuit et des substances nutritives, d'en bas à travers une colonne du détecteur qui contient une couche régulièrement active de microorganismes, caractérisé par le fait qu'ensuite la phase liquide est séparée de la phase gazeuse, et que la phase gazeuse est ramenée au pied de la colonne du détecteur à l'aide d'une pompe à gaz, en mesurant par une sonde à oxygène la teneur en O2 de la phase gazeuse du côté pression de la pompe et en enregistrant la valeur mesurée.

2. Procédé continu selon revendication 1, caractérisé par le fait que la couche de microorganismes dans la colonne du détecteur est chargée mécaniquement par un fort courant de gaz turbulent de manière qu'il se forme une bio-couche mince, régulièrement active.

3. Procédé continu selon revendications 1 à 2, caractérisé par les nourritures seront adjoutées d'une façon stérile et concentrée.

4. Procédé continu selon revendications 1 à 3, caractérisé par le fait que la colonne du détecteur est alimentée alternativement avec de l'eau à tester, tempérée à 34°Celsius oxygénée jusqu'à la saturation, et de l'eau traitée (p.ex. eau potable).

5. Procédé continu selon revendications 1 à 4, caractérisé par le fait que la consommation d'oxygène de la couche de microorganismes se trouve dans un état de balance dynamique grâce à l'affluence d'oxygène dans le milieu à tester et la compensation de pression avec l'atmosphère.

6. Procédé continu selon revendication 4, caractérisé par le fait que l'état de balance dynamique est réglé par la quantité de substances nutritives à env. 50% de la saturation d'oxygène, indiquant directement l'effet stimulant ou gênant du milieu à tester en tant que changement de l'état de balance.

7. Bio-moniteur à l'analyse continue d'eau, déterminant l'effet nuisible de substances nocives y contenues sur l'activité respiratoire de microorganismes, contenant
les unités pour tempérer et aérer, en vue de la saturation d'oxygène de l'eau à tester et du réglage de la température optimale pour les bactéries,
2 colonnes du détecteur, alternativement réglables, contenant une couche régulièrement active de microorganismes,
les dispositifs pour amener un fort courant de gaz turbulant au pied des colonnes du détecteur,
caractérisé par le fait qu'il existe des dispositifs pour séparer la phase liquide de la phase gazeuse à la tête des colonnes du détecteur, ainsi que des installations de tuyaux flexibles pour mener le courant de gaz séparé en circuit, avec une pompe à gaz et une sonde à oxygène pour mesurer la teneur en O2 du courant de gaz.

8. Bio-moniteur selon revendication 7, caractérisé par le fait que la compensation de pression avec l'atmosphère se produit à l'aide de deux trous de forage de 1 mm et de 2 mm de diamètre, situés dans le couvercle de fermeture du tuyau ascendant au bout supérieur des colonnes du détecteur.

9. Bio-moniteur selon revendications 6 à 7, caractérisé par le fait que l'espace entre les deux trous de forage dans le couvercle de fermeture des colonnes de détecteur est fourré de laine minérale ou d'une substance comparable de façon qu'il n'y ait qu'une compensation de pression ralentie entre l'intérieur du détecteur et l'atmosphère.
